Europäisches Patentamt

European Patent Office

Office européen des brevets

(11) Numéro de publication: **0 067 754**
**A2**

## (12) DEMANDE DE BREVET EUROPEEN

(21) Numéro de dépôt: **82400986.4**

(22) Date de dépôt: **27.05.82**

(51) Int. Cl.³: **A 61 B 6/02**
**A 61 B 6/06**

(30) Priorité: **10.06.81 BE 889159**

(43) Date de publication de la demande:
**22.12.82 Bulletin 82/51**

(84) Etats contractants désignés:
**DE FR IT NL**

(71) Demandeur: THOMSON-CSF
173, Boulevard Haussmann
F-75379 Paris Cedex 08(FR)

(72) Inventeur: Munch, Joseph
THOMSON-CSF SCPI 173, bld Haussmann
F-75379 Paris Cedex 08(FR)

(74) Mandataire: Barbin le Bourhis, Joel et al,
THOMSON-CSF SCPI 173, boulevard Haussmann
F-75379 Paris Cedex 08(FR)

(54) Dispositif pour entraîner en rotation une trame anti-diffusante dans un tomographe Rontgen.

(57) Le dispositif comporte en ordre principal une plaque de base 4 avec rainure de guidage annulaire 5, un élément de commande annulaire sans fin 6, librement placé et mobile dans la rainure de guidage 5 susmentionnée, des moyens de liaison 7 placés entre l'élément de commande annulaire 6 et la trame anti-diffusante 1 disposée au centre, et un moyen d'entraînement pour entraîner l'élément de commande annulaire 6 et la trame anti-diffusante y accouplée.

FIG_1

EP 0 067 754 A2

1

# DISPOSITIF POUR ENTRAINER EN ROTATION
## UNE TRAME ANTI-DIFFUSANTE
## DANS UN TOMOGRAPHE RONTGEN

L'invention a pour objet un dispositif pour entraîner en rotation à la périphérie et dans un plan horizontal une trame anti-diffusante sans axe central.

Il est bien connu que la commande du mouvement de rotation d'une telle trame anti-diffusante est nécessaire pour diriger les lamelles verticales, disposées l'une à proximité de l'autre, de telle manière qu'en direction longitudinale, elles courent toujours dans la direction du tube Röntgen qui se déplace circulairement au-dessus de la trame.

Pour arriver à ce résultat, on connaît un dispositif dans lequel la trame anti-diffusante est montée dans un disque ou anneau de soutien relativement grand qui, à sa périphérie est pourvu d'une chaîne ou courroie dentée dans laquelle un pignon de chaîne ou disque à courroie dentée entraîné en rotation, est en prise pour entraîner en rotation la trame anti-diffusante. Le disque est alors guidé à sa périphérie par de petites roues de guidage.

Ce dispositif a toutefois le désavantage que son prix de revient est relativement élevé et que son montage est relativement compliqué.

Pour obvier à ces inconvénients, le dispositif selon l'invention comporte en ordre principal une plaque de base avec rainure de guidage annulaire, un élément de commande annulaire sans fin librement placé et mobile dans la rainure de guidage susmentionnée, des moyens de liaison placés entre l'élément de commande annulaire et la trame anti-diffusante disposée au centre, et un moyen d'entraînement pour entraîner l'élément de commande annulaire et la trame anti-diffusante y accouplée.

Ce dispositif a l'avantage d'être composé de peu d'éléments. Son prix de revient est en outre très bas.

Il est décrit ci-après à titre d'exemple une forme d'exécution dudit dispositif conforme à l'invention, à savoir une forme d'exécution possible de celle-ci mais nullement limitée à la forme ci-après décrite. La présente description est accompagnée de dessins, dans lesquels :

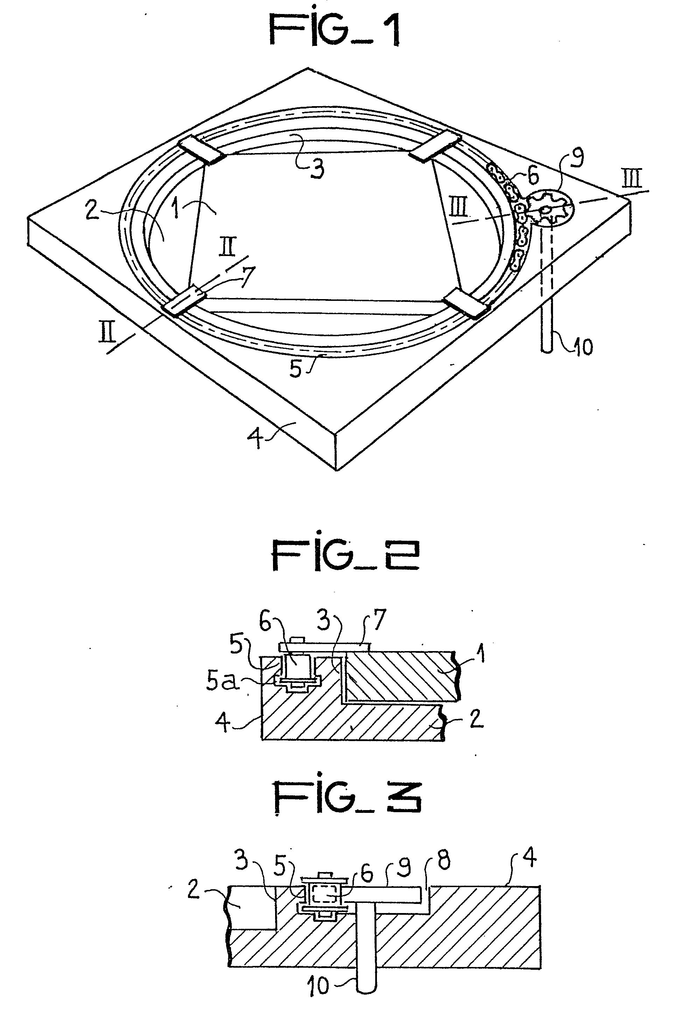

- la figure 1 est une vue en perspective du dispositif ;

- la figure 2 une coupe transversale suivant la ligne II-II de la figure 1 ;

- la figure 3 une coupe transversale suivant la ligne III-III de la figure 1 ;

Dans ces figures on remarque que le cadre carré de la trame anti-diffusante 1 se trouve dans un creux circulaire 2 pourvu d'une paroi verticale 3, d'une plaque de base 4 en matière plastique synthétique, dont le coefficient de frottement est très faible.

Les dimensions du creux circulaire 2 et du cadre de la trame anti-diffusante 1 sont choisies de telle manière que la trame anti-diffusante se trouve noyée dans le creux circulaire 2 et qu'elle puisse être entraînée dans ce creux. Autour du creux circulaire 2 il est prévu dans la plaque de base 4 une rainure annulaire 5, dont la section transversale a la forme d'un T renversé. Dans cette rainure annulaire, il est prévu avec un faible jeu, une chaîne articulée 6 sans fin, réunie par un maillon de liaison 7 en quatre endroits et à chaque angle du cadre de la trame 1 anti-diffusante. Dans la plaque de base et tout contre la rainure annulaire 5, il est prévu un évidement circulaire 8 qui, latéralement, aboutit dans la rainure annulaire 5 susmentionnée, dans laquelle il est logé un pignon de chaîne 9 qui est en prise avec la chaîne articulée 6 placée dans la rainure annulaire 5 et qui est monté sur un arbre de commande 10.

Pour monter le dispositif, on introduit dans la rainure annulaire 5, par l'évidement 8, la chaîne articulée 6; de telle manière que les maillons inférieurs de la chaîne articulée 6 se trouvent dans la partie élargie 5a de la rainure annulaire 5. Ainsi la chaîne articulée 6 , après avoir été mise en prise avec le pignon de chaîne 9, ne peut plus se dégager de la rainure annulaire 5, lorsque le pignon de chaîne 9 la fait tourner en rond dans la rainure 5 annulaire. Pendant ce mouvement, les maillons de liaison 7 tirent circulairement la trame 1 anti-diffusante dans le creux circulaire 2.

En utilisant ce moyen et en choisissant pour faire la plaque de base 4, la matière plastique synthétique qui convient, on arrive à maintenir le coefficient de frottement dans les limites admissibles, entre les éléments du dispositif.

Il va de soi que la forme et les dimensions des éléments ci-avant décrits, tout comme le montage de ceux-ci dans leurs positions respectives ainsi que le choix du matériau à utiliser pour les fabriquer peuvent différer, à condition de rester dans le cadre de l'invention.

R E V E N D I C A T I O N S

1. Dispositif pour entraîner en rotation une trame anti-diffusante dans un tomographe Röntgen, caractérisé par le fait qu'il comporte en ordre principal une plaque de base (4) avec rainure de guidage annulaire (5), un élément de commande annulaire sans fin (6) librement placé et mobile dans la rainure de guidage (5) susmentionnée, des moyens de liaison (7) placés entre l'élément de commande annulaire (6) et la trame anti-diffusante (1) disposée au centre, et un moyen d'entraînement pour entraîner l'élément de commande annulaire (6) et la trame anti-diffusante (1) y accouplée.

2. Dispositif conforme à la revendication 1, caractérisé par le fait que la rainure de guidage annulaire (5) a une section transversale en forme de T renversé, dans laquelle il est placé une chaîne articulée (6) sans fin dont les maillons sous-jacents courent dans la partie (5a) de la rainure de guidage annulaire (5)pour maintenir la chaîne articulée dans la rainure de guidage (5).

3. Dispositif conforme à la revendication 1, caractérisé par le fait que la plaque de base (4) est en matière plastique synthétique.

4. Dispositif conforme à la revendication 1, caractérisé par le fait que dans la plaque de base (4) il est prévu un creux circulaire dans lequel la trame anti-diffusante est noyée.

5. Dispositif conforme à la revendication 2, caractérisé par le fait que dans la plaque de base (4), il est prévu un évidement (8) de forme circulaire qui, latéralement, aboutit dans la rainure de guidage annulaire (5), pour introduire la chaîne articulée (6) dans cette rainure de guidage et loger un pignon de chaîne (9) en prise avec la chaîne articulée susmentionnée.

6. Dispositif conforme à la revendication 2, caractérisé par le fait que la trame anti-diffusante (1) est, à plusieurs endroits et à sa périphérie, reliée à la chaîne articulée (6) par des maillons de liaison (7).

0067754

1/1

FIG_1

FIG_2

FIG_3